(19) Europäisches Patentamt
European Patent Office
Office européen des brevets

(11) Veröffentlichungsnummer : **0 019 172**
**B1**

(12) # EUROPÄISCHE PATENTSCHRIFT

(45) Veröffentlichungstag der Patentschrift : **13.07.83**

(51) Int. Cl.³ : **C 07 D223/20**, C 07 D401/06, C 07 D403/04, A 61 K 31/55

(21) Anmeldenummer : **80102418.3**

(22) Anmeldetag : **05.05.80**

(54) **6-Substituierte 11-Alkylen-morphantridine, Verfahren zu ihrer Herstellung und diese enthaltende therapeutische Mittel.**

(30) Priorität : **10.05.79 DE 2918778**

(43) Veröffentlichungstag der Anmeldung : **26.11.80 Patentblatt 80/24**

(45) Bekanntmachung des Hinweises auf die Patenterteilung : **13.07.83 Patentblatt 83/28**

(84) Benannte Vertragsstaaten : **AT BE CH DE FR GB IT LI LU NL SE**

(56) Entgegenhaltungen :
**DE A 2 006 674**
**DE A 2 633 782**
**DE B 2 035 517**
**GB A 1 207 116**
**US A 3 497 596**

**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Steiner, Gerd, Dr.**
**Oberer Waldweg 1**
**D-6719 Kirchheim (DE)**
Erfinder : **Franke, Albrecht, Dr.**
**Mandelring 11**
**D-6706 Wachenheim (DE)**
Erfinder : **Lenke, Dieter, Dr.**
**Kekuleplatz 1**
**D-6700 Ludwigshafen (DE)**
Erfinder : **Teschendorf, Hans-Juergen, Dr.**
**Rene-Bohn-Strasse 4**
**D-6700 Ludwigshafen (DE)**
Erfinder : **Worstmann, Wolfgang, Dr.**
**Am Bildstock 11**
**D-6718 Gruenstadt 1 (DE)**
Erfinder : **Kreiskott, Horst, Dr.**
**Am Boehlig**
**D-6706 Wachenheim (DE)**

**EP 0 019 172 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# 6-substituierte 11-Alkylen-morphantridine, Verfahren zu ihrer Herstellung und diese enthaltende therapeutische Mittel

Die Erfindung betrifft bestimmte in 6-Stellung substituierte 11-Alkylen-morphantridine, die man auch als 11-Alkylen-dibenzo [b,e]-azepine bezeichnet, Verfahren zu ihrer Herstellung und diese enthaltende Arzneimittel.

Es ist bekannt, daß tricyclische Ringsysteme mit einer Dibenzo-struktur zu einem zentralen heterocyclischen 7-Ring, der gegebenenfalls einen basischen Seitenrest, wie beispielsweise einen N-Methylpiperazinrest aufweist, neuroleptische Wirkungen aufweisen können. Solche Tricyclen sind beispielsweise N-Methylpiperazin-Derivate von Dibenzo [b,e]-[1,4]-diazepinen (Clozapine) oder Morphantridinen (Perlapine), wie beispielsweise aus der Zusammenfassung von J. Schmutz in der Arzneimittelforschung 25, 712-720 (1975) hervorgeht. Aus der GB-A 1 207 116 und der DE-B 2 035 517 sind beispielsweise Morphantridine mit einem Alkylenaminrest in 11-Stellung bekannt, für die u. a. eine Antihistamin-, sedative, spasmolytische, psychotrope und/oder antiemetische Wirkung beschrieben wird. Die gefundenen Wirkungen befriedigen nicht immer.

Ziel der Erfindung ist die Bereitstellung neuer Verbindungen mit besseren Wirksamkeiten und geringeren Nebenwirkungen.

Es wurde nun gefunden, daß 6-substituierte 11-Alkylen-morphantridine der allgemeinen Formel I

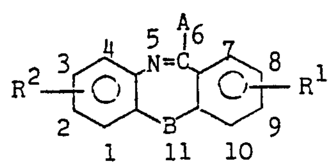

(I)

in der $R^1$ und $R^2$ Wasserstoff, Fluor, Chlor oder Methyl bedeuten, A für 4-Methyl-piperazin-1-yl, 4-Ethylpiperazin-1-yl, 4-Methyl-4-oxy-piperazin-1-yl, N'-Methyl-homopiperazin-1-yl oder 2-Piperidin-1-yl-ethylamino steht und B den Alkylenrest

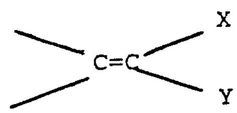

in dem X ein Wasserstoffatom und Y eine Cyanogruppe bedeuten, die reinen cis- und trans-Isomeren und die physiologisch verträglichen Säureadditionssalze wertvolle pharmakologische Eigenschaften aufweisen.

Es wird darauf hingewiesen, daß die erfindungssgemäßen Verbindungen der Formel I als cis-trans-Isomere Ia und b vorliegen.

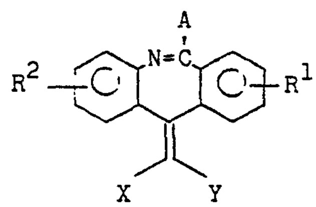

(Ia)

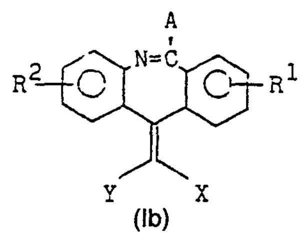

(Ib)

Gegebenenfalls können die cis-trans-Isomeren beispielsweise durch fraktionierte Kristallisation oder durch Säulenchromatographie getrennt werden. Die Zuordnung der einzelnen Isomeren erfolgt beispielsweise durch Röntgenstrukturanalyse, wie aus den Beispielen hervorgeht.

Aufgrund der oben genannten Bedeutungen sind die folgenden Verbindungen als besonders wirksam zu nennen :

cis, trans-11-Cyanmethylen-6-(4-methyl-piperazin-1-yl)-morphantridin,
cis-11-Cyanmethylen-6-(4-methyl-piperazin-1-yl)-morphantridin,
trans-11-Cyanmethylen-6-(4-methyl-piperazin-1-yl)-morphantridin,
cis,trans-11-Cyanmethylen-6-(4-methyl-4-oxy-piperazin-1-yl)-morphantridin,
cis-11-Cyanmethylen-6-(4-methyl-4-oxy-piperazin-1-yl)-morphantridin,
trans-11-Cyanmethylen-6-(4-methyl-4-oxy-piperazin-1-yl)-morphantridin,
cis,trans-11-Cyanmethylen-2-methyl-6-(4-methyl-piperazin-1-yl)-morphantridin,
cis,trans-11-Cyanmethylen-3-methyl-6-(4-methyl-piperazin-1-yl)-morphantridin,

cis-11-Cyanmethylen-3-methyl-6-(4-methyl-piperazin-1-yl)-morphantridin,
trans-11-Cyanmethylen-3-methyl-6-(4-methyl-piperazin-1-yl)-morphantridin,
cis,trans-11-Cyanmethylen-6-(4-ethyl-piperazin-1-yl)-morphantridin,
trans-11-Cyanmethylen-6-(4-ethyl-piperazin-1-yl)-morphantridin,
cis,trans-11-Cyanmethylen-6(N'-methyl-homopiperazin-1-yl)-morphantridin,
cis,trans-11-Cyanmethylen-6-(2-piperidin-1-yl-ethylamino)-morphantridin.

Wie die Ausführungsbeispiele zeigen, kann im Einzelfall die Trennung in das cis- und trans-Isomere ohne allzu großen Aufwand vorgenommen werden.

Die erfindungsgemäßen Verbindungen der Formel I werden hergestellt, indem man eine Verbindung der Formel II

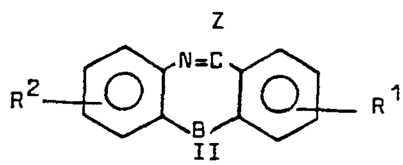

in der $R^1$, $R^2$ und B die für Formel I angegebenen Bedeutungen haben und Z eine nukleofuge Abgangsgruppe darstellt, mit einem Nukleophil AH, in dem A die für Formel I angegebenen Bedeutungen hat, umsetzt, gegebenenfalls in das reine cis- und trans-Isomere trennt und gegebenenfalls im Falle eines 4-Methyl-piperazin-1-yl-restes die erhaltene Verbindung in das N-Oxid und/oder in das Säureadditionssalz einer physiologisch verträglichen Säure überführt.

Als nukleofuge Abgangsgruppe für Z kommen Halogenatome, insbesondere Chlor, Sulfhydril, ein niederer Alkoxy-, Alkylthio- oder Alkylaminorest mit 1 bis 3 C-Atomen im Alkyl, der p-Nitrobenzylthio- oder Tosyloxy-rest in Betracht. Davon ist besonders bevorzugt ein Chloratom.

Die Umsetzung erfolgt zweckmäßig in Gegenwart einer überschüssigen Menge des verwendeten Amins AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Ether, insbesondere Tetrahydrofuran oder Dioxan, von Benzol oder eines Benzolkohlenwasserstoffes, wie Xylol, Mesitylen oder Decahydronaphthalin gearbeitet werden. Die Umsetzung erfolgt in der Regel bei Temperaturen von 80 bis 150 °C, bevorzugt 90 bis 120 °C, und ist im allgemeinen innerhalb von 3 bis 10 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2-fachem molarem bis 20-molarem Überschuß verwendet.

Falls die nukleofuge Gruppe Z einen Alkylaminorest darstellt, ist die Gegenwart einer katalytischen Menge einer starken Säure, z. B. p-Toluolsulfonsäure oder Schwefelsäure, von Vorteil.

Die Überführung einer Verbindung der Formel I in das N-Oxid erfolgt in üblicher Weise, zweckmäßig mit wäßrigem Wasserstoffperoxid (30 Gew.%) in ethanolischer Lösung. Die Überführung in das Säureadditionssalz einer physiologisch verträglichen Säure erfolgt ebenso in üblicher Weise.

Die Ausgangsverbindungen der Formel II werden erhalten, indem man ein 5,6-Dihydro-11-alkylen-morphantridin-6-on der Formel III

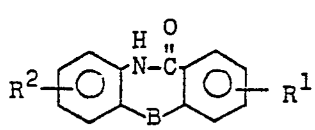
(III)

in der $R^1$, $R^2$ und B die für Formel II angegebenen Bedeutungen haben, in an sich üblicher Weise mit überschüssigem Phosphoroxychlorid, das gleichzeitig als Lösungsmittel dient, und gegebenenfalls in Gegenwart einer katalytischen Menge N,N-Dimethylanilin 3 bis 6 Stunden auf Rückflußtemperaturen erhitzt und das erhaltene Iminochlorid nach dem Abdestillieren des überschüssigen Phosphoroxychlorids und Aufarbeitung in einem wäßrigen zweiphasigen System durch Extraktion mit einem chlorierten Kohlenwasserstoff, wie Chloroform oder Methylenchlorid, isoliert und gegebenenfalls mit einem weiteren Nukleophil ZH, in dem Z die für die Formel II angegebenen Bedeutungen hat, in an sich üblicher Weise umsetzt.

Das neue 5,6-Dihydro-11-alkylen-morphantridin-6-on der Formel III, in der $R^1$, $R^2$ und B die für Formel I angegebenen Bedeutungen haben, wird durch Carbonyl-Olefinierung hergestellt, in dem man ein 5,6-Dihydro-morphantridin-6,11-dion der Formel IV

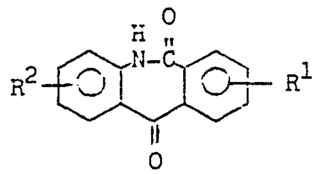
(IV)

in der $R^1$ und $R^2$ die für Formel I angegebenen Bedeutungen haben, mit einem Phosphonat der Formel V

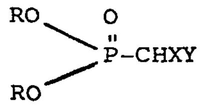
(V)

in der R einen Alkylrest mit 1 bis 3 C-Atomen und X und Y die für Formel III angegebenen Bedeutungen haben, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80 °C umsetzt oder mit einem Phosphoniumsalz der Formel VI

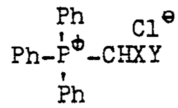
(VI)

in der Ph für einen Phenylrest steht und X und Y die für Formel V angegebenen Bedeutungen haben, unter den Bedingungen der klassischen Wittig-Reaktion in einem aprotischen organischen Lösungsmittel insbesondere einem gesättigten aliphatischen oder gesättigten cyclischen Ether, wie Diethylether, Tetrahydrofuran oder Dioxan, oder vorzugsweise in Dimethylformamid, in Gegenwart von einem Moläquivalent einer Base, insbesondere einem Alkalialkoholat, vorzugsweise Natriummethylat oder Natriumethylat oder Natriumhydrid, Natriumamid oder einer metallorganischen Verbindung, wie Butyllithium, bei Temperaturen von 20 bis 100 °C umsetzt.

Ein weiteres Verfahren zur Herstellung der Verbindungen der Formel I ist dadurch gekennzeichnet, daß man eine Verbindung der Formel VII

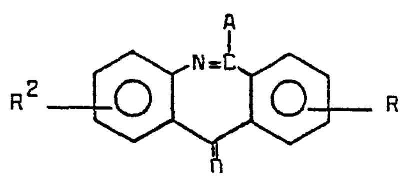
(VII)

in der $R^1$, $R^2$ und A die für Formel I angegebenen Bedeutungen haben, mit einer Verbindung der Formel V

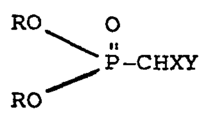
(V)

in der X und Y die im Rest B der Formel I für X und Y angegebenen Bedeutungen haben und R Alkylreste mit 1 bis 3 C-Atomen bedeuten, unter den Bedingungen der Wittig-Horner-Reaktion umsetzt und gegebenenfalls im Falle eines 4-Methyl-piperazin-1-yl-restes die erhaltene Verbindung in das N-Oxid und/oder Säureadditionssalz einer physiologisch verträglichen Säure überführt.

An sich übliche Bedingungen der Wittig-Horner-Reaktion sind ein inertes Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80 °C.

Die 5,6-Dihydro-morphantridin-6,11-dione der Formel IV sind teilweise literaturbekannt (F. Hunziker et al., Helv. Chim. Acta 49, 1433-1439 (1966) ; L. H. Werner et al., J. Med. Chem. 8, 74-80 (1965) ; G. Caronna et al., Gazz. chim. ital. 84, 1135-1140 (1954)) oder sind aus den entsprechenden Anthrachinonen durch Ringerweiterung mit Hilfe der Schmidt-Reaktion, wie es in den Beispielen beschrieben wird, oder durch Halogensubstitution des Grundkörpers (E. Hardtmann u. H. Ott, J. Org. Chem. 34, 2244-2248 (1969)) zugänglich.

Neben den in den Beispielen aufgeführten Verbindungen seien weiterhin folgende Verbindungen beispielhaft genannt :

cis,trans-2-Chlor-11-cyanmethylen-6-(4-methyl-piperazin-1-yl)-morphantridin,
cis,trans-11-Cyanmethylen-8-methyl-6-(4-methyl-piperazin-1-yl)-morphantridin,

cis,trans-11-Cyanmethylen-9-methyl-6-(4-methyl-piperazin-1-yl)-morphantridin,
cis,trans-11-Cyanmethylen-3-fluor-6-(4-methyl-piperazin-1-yl)-morphantridin,
cis,trans-11-Cyanmethylen-8-fluor-6-(4-methyl-piperazin-1-yl)-morphantridin.

Die erfindungsgemäßen Verbindungen der Formel I werden in der Regel in Form von gelblichen bis gelben Kristallen erhalten und können durch Umkristallisation aus den üblichen organischen Lösungsmitteln, bevorzugt aus einem niederen Alkohol, wie Ethanol, Umkristallisiert oder durch Säulenchromatographie gereinigt werden.

Falls erforderlich erfolgt eine Trennung in den einzelnen cis- oder trans-Isomeren durch fraktionierte Kristallisation in einem chlorierten Kohlenwasserstoff, bevorzugt Methylenchlorid, einem niederen einwertigen Alkohol, bevorzugt Methanol oder Ethanol, oder einem gesättigten cycloaliphatischen Kohlenwasserstoff, bevorzugt Cyclohexan, oder durch Säulenchromatographie, insbesondere an Kieselgel, mit Methylenchlorid oder einer Mischung aus Methylenchlorid und Methanol im Verhältnis 99 : 1 bis 85 : 15 Volumenteilen. Die fraktionierte Kristallisation wird besonders bevorzugt mit zwei Lösungsmitteln durchgeführt.

Die freien 6-substituierten 11-Alkylen-morphantridine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Maleinsäure und Methansulfonsäure oder können beispielsweise dem Journal of Pharmaceutical Sciences, Volume 66, Seiten 1 bis 5 (1977) entnommen werden.

Nach den Ergebnissen der pharmakologischen Experimente eignen sich die erfindungsgemäßen Substanzen in Anbetracht ihrer sedativen, apomorphin-antagonistischen, analgetischen, reserpin-antagonistischen oder anticholinergischen Wirkung als Neuroleptika, Sedativa, Hypnotika, Analgetika, Antidepressiva oder Antiparkinsonmittel.

Zur Wirkungsanalyse fanden folgende Methoden Verwendung :

1. Sedative Wirkung

Gruppen von 4 × 3 oder 8 × 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min.

Als ED 50 % wird die Dosis bestimmt, welche im Vergleich zu unbehandelten Kontrolltieren eine Abnahme der Orientierungshypermotilität um 50 % bewirkt.

2. Analgetische Wirkung

Die analgetische Wirkung wird mit Hilfe der sogenannten Brennstrahl-Methode nach D'Amour und Smith (1941) bestimmt. Dabei erhalten Gruppen von 10 weiblichen NMRI-Mäusen die Substanzen intraperitoneal appliziert. Die Schmerzreaktion wird 30 min nach der Applikation ausgelöst. Gemessen wird die Reaktionszeit bis zum Wegziehen des Schwanzes nach Bestrahlung mit einem fokussierten Lichtbündel.

Als ED 100 % wird die Dosis bestimmt, welche im Vergleich zu einer Kontrollgruppe die Reaktionszeit um 100 % verlängert.

3. Anticholinergische Wirkung

Gruppen von 10 weiblichen NMRI-Mäusen erhalten Physostigmin in einer letalen Dosis (0,825 mg/kg) subcutan. Die Prüfsubstanzen werden 30 min vor der Physostigminapplikation oral appliziert.

Als ED 50 wird die Substanzdosis bestimmt, welche 50 % der Tiere vor dem Tod durch Physostigmin schützt.

4. Apomorphin-antagonistische Wirkung

An Gruppen von 4 bis 6 weiblichen Sprague-Dawley-Ratten werden durch subcutane Applikation von 1,5 mg/kg Apomorphin Kieferbewegungen ausgelöst, die über implantierte Elektroden registriert werden (Mandibulogramm nach Kubacki, 1978).

Als ED 50 % wird die Dosis bestimmt, welche die Anzahl der Kieferbewegungen im Vergleich zu unbehandelten Kontrolltieren um 50 % reduziert.

5. Akute Toxizität

Gruppen von 5 bis 10 weiblichen NMRI-Mäusen erhalten die Substanzen i.p. Als LD 50 wird die Dosis ermittelt, nach welcher 50 % der behandelten Tiere sterben.

In diesen Experimenten (Tab. 1) werden bei den Verbindungen Beispiel 1 (cis-trans-Gemisch und cis-Isomeres), Beispiel 7a (cis-trans-Gemisch und cis-Isomeres), Beispiel 7b (cis-trans-Gemisch), Beispiel 8

(cis-trans-Gemisch) und Beispiel 10 (cis-trans-Gemisch) starke sedative Wirkungen beobachtet, die in der Größenordnung der Referenzsubstanzen Clozapin oder Perlapin liegen oder diese übertreffen.

Eine analgetische Wirkung findet man bei Beispiel 7a (cis-trans-Gemisch und cis-Isomeres). Das cis-Isomere ist deutlich aktiver als Clozapin.

Die am Physostigmin-antagonismus beobachtet anticholinergische Wirkung tritt besonders bei den Verbindungen Beispiel 1 (cis-trans-Gemisch und trans-Isomeres), Beispiel 7b (cis-trans-Gemisch), Beispiel 8 (cis-trans-Gemisch und trans-Isomeres), Beispiel 9 (cis-trans-Gemisch), Beispiel 11 (cis-trans-Gemisch und trans-Isomeres) in Erscheinung. Bei Beispiel 1 (cis-trans-Gemisch), Beispiel 7b (cis-trans-Gemisch) und Beispiel 8 (cis-trans-Gemisch) ist sie ähnlich wie beim Clozapin im Verein mit stärkeren sedativen Effekten (s.o.) zu beobachten.

Zusammen mit den genannten Effekten tritt bei den meisten Verbindungen auch eine für Neuroleptika typische apomorphin-antagonistische Wirkung auf, die sich ebenfalls bei den Referenzsubstanzen findet.

Vergleicht man die pharmakologischen Eigenschaften der vorliegenden cis-trans-Isomerengemische mit denen der einzelnen reinen Isomeren, so ergeben sich überraschenderweise nicht nur quantitative, sondern auch qualitative Differenzen, so daß bei verschiedenen Substanzen neuartige und interessante Wirkungskombinationen gefunden werden.

Das Wirkprofil des cis-trans-Gemisches Beispiel 1 ähnelt dem der Referenzsubstanz Clozapin. Es wirkt aber stärker sedativ und anticholinergisch und ist nicht analgetisch aktiv. Die apomorphin-antagonistische Wirkung ist etwas schwächer als beim Clozapin.

Das cis-Isomere von Beispiel 1 ist Träger der sedativen Wirkung bei einer etwa dem Clozapin vergleichbaren anticholinergischen und geringerer apomorphin-antagonistischer Wirkung.

Das trans-Isomere von Beispiel 1 hingegen zeichnet sich besonders durch anticholinergische und apomorphin-antagonistische Wirkung aus. Die sedative Wirkung tritt ganz zurück. Ein derartiger Wirkungstyp ist neu und sowohl von dem des Clozapin als auch des Perlapin eindeutig abzugrenzen.

Auch die trans-Isomeren von Beispiel 7a, 8 und 11 wirken nicht oder nur wenig sedativ, stärker anticholinergisch und apomorphin-antagonistisch und sind ebenso wie das trans-Isomere von Beispiel 1 von den jeweiligen Isomerengemischen zu unterscheiden.

Ein weiteres Isomerengemisch Beispiel 7a wirkt bei hoher sedativer Aktivität (> Clozapin und Perlapin) mäßig anticholinergisch und stärker analgetisch. Verantwortlich für die starke sedative und die starke analgetische Wirkung ist das cis-Isomere. Auch diese Verbindung stellt in der Kombination sedative plus analgetische Wirkung bei fehlender anticholinergischer und schwacher apomorphin-antagonistischer Wirkung im Vergleich zum Clozapin und Perlapin einen neuen Wirkungstyp dar.

(Siehe die Tabelle 1, Seite 7)

Tabelle 1

| Beisp. Nr. | Geometrische Isomerie | Sedative Wirkung ED 50 % | Sedative Wirkung R.W. [1] | Analgetische Wirkung ED 100 % | Analgetische Wirkung R.W. | Apomorphin-Antagonismus ED 50 % | Apomorphin-Antagonismus R.W. | Anticholinergische Wirkung ED 50 % | Anticholinergische Wirkung R.W. | Toxizität LD 50 |
|---|---|---|---|---|---|---|---|---|---|---|
| 1 | cis-trans-G.[2] | 2,78 | 1,71 | 46,4 | 0,04 | 22 | 0,37 | 6,26 | 2,25 | 121 |
| | cis-Isomeres | 1,98 | 2,39 | 31,6 | 0,07 | 46 | 0,17 | 17,4 | 0,81 | 147 |
| | trans-Isomeres | > 21,5 | < 0,22 | > 21,5 | < 0,10 | 12 | 0,67 | 4,7 | 3,00 | 332 |
| 7a | cis-trans-G. | 0,804 | 5,90 | 5,17 | 0,40 | 35 | 0,23 | 31,2 | 0,45 | 178 |
| | cis-Isomeres | 1,06 | 4,47 | 0,740 | 2,18 | 85 | 0,09 | 100 | 0,14 | — |
| | trans-Isomeres | 33,6 | 0,14 | 68,1 | 0,03 | 19 | 0,42 | 12,3 | 1,15 | — |
| 7b | cis-trans-G. | 3,35 | 1,41 | 10,0 | 0,21 | 42 | 0,19 | 2,74 | 5,15 | 50,0 |
| 8 | cis-trans-G. | 3,34 | 1,42 | > 10,0 | < 0,21 | 43 | 0,19 | 7,80 | 1,81 | 100 |
| | trans-Isomeres | > 100 | < 0,04 | > 46,4 | < 0,04 | 22 | 0,37 | 5,60 | 2,52 | — |
| 9 | cis-trans-G. | > 21,5 | < 0,22 | 46,4 | 0,04 | > 100 | < 0,08 | 5,49 | 2,57 | 69,8 |
| 10 | cis-trans-G. | 1,61 | 2,94 | > 10,0 | < 0,21 | 46 | 0,17 | > 10,0 | < 1,41 | 5,35 |
| 11 | cis-trans-G. | 14,3 | 0,33 | > 46,4 | < 0,04 | 85 | 0,09 | 1,98 | 7,12 | 464 |
| | cis-Isomeres | 17,6 | 0,26 | > 46,4 | < 0,04 | > 100 | < 0,08 | 100 | 0,14 | — |
| | trans-Isomeres | 100 | 0,04 | > 100 | < 0,02 | 25 | 0,32 | 3,74 | 3,77 | 559 |
| Clozapin | | 4,74 | 1,00 | 2,08 | 1,00 | 8 | 1,00 | 14,1 | 1,00 | 215 |
| Perlapin | | 2,05 | 2,31 | > 21,5 | < 0,10 | 22 | 0,37 | > 21,5 | < 0,66 | 215 |

[1] R.W. = Relative Wirksamkeit
[2] G. = Gemisch

Die Erfindung betrifft dementsprechend auch ein therapeutisches Mittel, gekennzeichnet durch einen Gehalt an einer Verbindung der Formel I, deren reines cis- oder trans-Isomeres oder deren pharmakologisch verträgliches Säureadditionssalz als Wirkstoff neben üblichen Träger- und Verdünnungsmitteln.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden. Als Einzeldosen beim Menschen kommen 10 bis 100 mg in Betracht.

Es werden die üblichen galenischen Applikationsformen, fest oder flüssig, wie Tabletten, Kapseln, Pulver, Granulate, Dragees oder Lösungen verwendet. Diese werden in üblicher Weise, insbesondere durch Vermischen, hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polycinylpyrrolidon, wäßrigen oder nicht wäßrigen Trägern, Netzmitteln, Dispergiermitteln, Emulatoren und/oder Konservierungsmitteln, verarbeitet werden (vgl. L.G. Goodman, A. Gilman, The Pharmacological Basis of Therapeutics). Die so erhaltenen Präparate enthalten den Wirkstoff normalerweise in einer Menge von 0,001 und 99 Gew.%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Die folgenden Beispiele dienen zur Erläuterung der Erfindung :

## Beispiel 1

cis- und trans-11-Cyanmethylen-6-(4-methyl-piperazin-1-yl)-morphantridin

a) 20,0 g (81 mM) 11-Cyanmethylen-5,6-dihydro-morphantridin-6-on (cis, trans-Isomerengemisch) wird mit 160 ml Phosphoroxychlorid und 3,5 ml N,N-Dimethylanilin versetzt und 4 Stunden unter Stickstoffatmosphäre am Rückfluß gekocht. Nach vollständigem Abdestillieren des überschüssigen Phosphoroxychlorids und Dimethylanilins am Ölpumpenvakuum verteilt man den Rückstand zwischen Methylenchlorid und Wasser, extrahiert die wäßrige Phase noch zweimal mit Methylenchlorid und wäscht die vereinigten organischen Phasen mit verdünnter HCL und Wasser gründlich nach. Trocknen und Einengen der organischen Phase liefert 20,8 g (97 %) 6-Chlor-11-cyanmethylen-morphantridin, das für die weitere Umsetzung genügend rein ist.

20,8 g (79 mM) 6-Chlor-11-cyanmethylen-morphantridin wird mit 60 ml N-Methyl-piperazin versetzt und 3 bis 5 Stunden bei 110 °C unter Stickstoff gerührt. Das dunkle homogene Reaktionsgemisch gießt man dann nach dem Abkühlen auf Eiswasser und saugt das gelbliche Rohprodukt, 11-Cyanmethylen-6-(4-methyl-piperazin-1-yl)-morphantridin, ab. Nach dem Trocknen im Vakuumtrockenschrank kristallisiert man das Rohprodukt aus Äthanol unter Zusatz von Aktivkohle um. Man erhält 19,5 g (75 %) gelbes 11-Cyanmethylen-6-(4-methyl-piperazin-1-yl)-morphantridin in Form eines cis,- trans-Isomerengemisches mit dem Schmp. 148-150 °C.

Zur Trennung der cis, trans-Isomeren digeriert man das Isomerengemisch in ca. 80 ml siedendem Methanol und saugt die unlöslichen Anteile in der Hitze ab. Man erhält 3,1 g gelbe Festkörper, die nach Ausweis des Dünnschichtchromatogramms (Kieselgel, Laufmittel Toluol/Methanol 85/15) vornehmlich aus dem unpolaren Isomeren a bestehen. Nach dem Einengen des Filtrats nimmt man den Rückstand in wenig siedendem Methylenchlorid auf, bis gerade alles in Lösung geht. Beim Abkühlen kristallisieren 3, 0 g gelbes Produkt aus, das schnell abgesaugt und mit sehr wenig eiskaltem Methylenchlorid nachgewaschen wird. Die Dünnschichtchromatographie zeigt eine sehr gute Anreicherung des polaren Isomeren b.

Durch mehrfache Wiederholung dieser beiden aufeinanderfolgenden Operationen erhält man insgesamt je ca. 10 bis 11 g Fraktionen der stark angereicherten cis, trans-Isomeren a und b, die dann noch 1 bis 2 mal aus Äthanol umkristallisiert werden.

Das reine Isomere a fällt in Form von gelben, rechteckigen Plättchen mit Schmp. 210 bis 212 °C, das reine Isomere b in Form von gelben spitzen Nadeln mit Schmp. 182 bis 184 °C an.

Die Röntgenstrukturanalyse zeigt, daß es sich bei a um das cis-isomere und bei b um das trans-isomere 11-Cyanmethylen-6-(4-methyl-piperazin-1-yl)-morphantridin handelt.

(Siehe die Schemas, Seite 9)

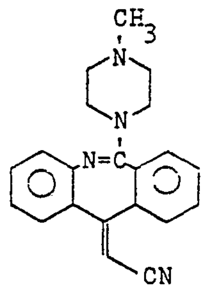

(a)

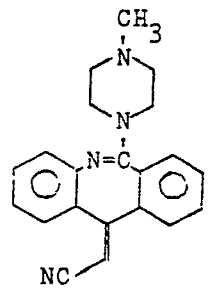

(b)

Zur Herstellung des Vorproduktes 11-Cyanmethylen-5,6-dihydro-morphantridin-6-on führt man eine Carbonyl-Olefinierung des 5,6-Dihydro-morphantridin-6,11-dions mit Hilfe der Wittig-Horner-Reaktion oder über die klassische Wittig-Synthese durch :

b) 30,0 g (135 mM) 5,6-Dihydro-morphantridin-6,11-dion werden in 300 ml Dimethylformamid gelöst und unter Stickstoff gerührt. Man läßt dann gleichzeitig 35,4 g (200 mM) Diäthyl-cyanmethyl-phosphonat und 35,0 g (200 mM) Natriummethylat (30 %) gelöst in 100 ml Dimethylformamid langsam zutropfen (Farbzunahme und Temperaturerhöhung zeigen den Beginn der Wittig-Reaktion an). Nach 12-stündigem Nachrühren bei Raumtemperatur gießt man das Reaktionsprodukt auf Eiswasser und saugt die ausfallenden Festkörper ab. Nach gutem Nachwaschen mit Wasser wird das Rohprodukt getrocknet und aus Äthanol umkristallisiert. Ausbeute : 32,5 g (98 %) 11-Cyanmethylen-5,6-dihydro-morphantridin-6-on, farblose Kristalle mit Schmp. 221-223 °C.

Beispiel 2

cis,trans-9-Chlor-11-cyanmethylen-6-(4-methyl-piperazin-1-yl)-morphantridin

a) 9-Amino-5,6-dihydro-morphantridin-6,11-dion

20,0 g (90 mM) 2-Aminoanthrachinon werden in einer Mischung aus 96 ml conz. Schwefelsäure und 32 ml Methylenchlorid eingetragen und unter Rühren bei Raumtemperatur gelöst. In diese Reaktionsmischung gibt man bei 20 °C (Kühlung von außen durch ein Wasserbad) portionsweise während 5 Stunden 6,8 g (105 mM) Natriumazid hinzu. Man läßt über Nacht bei Raumtemperatur rühren und gießt dann die Reaktionsmischung vorsichtig auf 3 l Eiswasser. Man stellt den pH-Wert der wäßrigen Mischung mit conz. Natronlauge auf 9, saugt die ausfallenden Festkörper ab und wäscht mit reichlich Wasser nach. Das Rohprodukt wird im Vakuum-Trockenschrank bei 70 °C getrocknet.

Zur Trennung des Isomerengemisches — es handelt sich nach Ausweis des 270 MHz $^1$H-NMR-Spektrums um 4 Aminoisomere — digeriert man in 1 l siedendem Äthanol und saugt den unlöslichen Anteil (ca. 1/4 der Gesamtmenge) in der Hitze ab. Man isoliert 4,5 g (21 %) stark angereichertes 9-Amino-5,6-dihydro-morphantridin-6,11-dion vom Schmp. 295-297 °C ; Umkristallisieren aus ca. 200 ml Äthanol/Dimethylformamid 3 : 1 unter Verwendung von Aktivkohle liefert das reine Isomere. Die Stellung der Amino-Gruppe ergiebt sich aus der Röntgenstrukturanalyse des Endproduktes (s. unten).

$^1$H-NMR (270 MHz, $D_6$DMSO) : $\delta$ = 6,30 (s ; $NH_2$), 6,97 (d ; 1H), 7,01 (s ; 1H), 7,20 (t, 1H), 7,37 (d, 1H), 7,59 (t, 1H), 7,72 (d, 1H), 7,98 (d, 1H), 10,70 (s, NH).

Durch fraktionierte Kristallisationen der äthanolischen Mutterlauge kann man die noch verbleibenden 2-, 3- und 8-Amino-5,6-dihydro-morphantridin-6,11-dione anreichern (Analyse der Fraktionen jeweils durch 270 MHz $^1$H-NMR Aufnahmen).

b) 9-Chlor-5,6-dihydro-morphantridin-6,11-dion

3,0 g (12,6 mM) 9-Amino-5,6-dihydro-morphantridin-6,11-dion werden in eine Mischung aus 120 ml Wasser und 120 ml conz. Salzsäure eingetragen. Bei 0 bis 5 °C tropft man unter gutem Rühren eine Lösung von 0,87 g (12,6 mM) Natriumnitrit in 10 ml $H_2O$ hinzu und läßt 2,5 Stunden bei einer Temperatur zwischen 0 und 5 °C nachrühren. Anschließend gibt man zur Zerstörung der überschüssigen salpetrigen Säure etwas Harnstoff hinzu und fügt darauf 120 mM eines frisch hergestellten Cu-I-Chlorid-Katalysators in conz. Salzsäure hinzu (Stickstoffentwicklung). Man läßt 30 Minuten bei Raumtemperatur nachrühren und erhitzt anschließend 1 Stunde auf 100 °C unter beständigem Rühren. Das Reaktionsgemisch wird nach dem Abkühlen auf Eiswasser gegossen und dreimal mit je 300 ml Methylenchlorid extrahiert. Die organische Phase wird anschließend mit Wasser gewaschen, getrocknet und eingeengt. Man isoliert 1,9 g 9-Chlor-5,6-dihydro-morphantridin-6,11-dion vom Schmp. 265-267 °C.

c) Weiterreaktion zum Endprodukt analog Beispiel 1 :

cis,trans-9-Chlor-11-cyanmethylen-5,6-dihydro-morphantridin-6-on nach Beispiel 1b : Schmp. 250-255 °C.

cis,trans-9-Chlor-11-cyanmethylen-6-(4-methyl-piperazin-1-yl)-morphantridin · 0,5 $H_2O$ nach Beispiel 1a : Schmp. 90-95 °C.

Zur Trennung der cis,trans-Isomeren kristallisierte man fraktioniert aus Äthanol um : als schwerer lösliche Fraktion kristallisiert zuerst das reine cis-Isomere (unpolarere Komponente auf der Dünnschichtplatte : Kieselgel, Laufmittel Toluol/Methanol 85/15) mit dem Schmp. 173-174 °C.

Die 9-Stellung des Chlors ist durch Röntgenstrukturanalyse des cis-Isomeren bewiesen.

d) cis,trans 3- bzw. 8-Chlor-11-cyanmethylen-6-(4-methyl-piperazin-1-yl)-morphantridin.

Das als Ausgangsmaterial eingesetzte, durch Ringerweiterung von 2-Chlor-anthrachinon nach Lit. I (L. H. Werner et al., J. Med. Chem. *8*, 74 (1965)) erhaltene Monochlor-5,6-dihydro-morphantridin-6,11-dion-Isomerengemisch (im wesentlichen sind 3 verschieden chlorierte Isomere vorhanden) konnte im Gegensatz zur Aussage von Lit. I durch fraktionierte Kristallisation nicht getrennt werden. (Es wurden zwar Fraktionen mit ähnlichem Schmp. wie in Lit. I angegeben erhalten, die aber nach Ausweis des 270 MHz $^1$H-NMR-Spektrums Gemische aus 2 bis 3 Isomeren waren). Deshalb wurde mit dem Isomerengemisch weitergearbeitet und erst auf der letzten Stufe eine Separierung vorgenommen.

Synthese analog Beispiel 1 :

Die Carbonyl-Olefinierung liefert ein Monochlor-11-cyanmethylen-5,6-dihydro-morphantridin-6-on-Isomerengemisch mit Schmp. 148-151 °C. Das im wesentlichen aus 6 Isomeren (Dünnschichtchromatographie, Kieselgel, Toluol/Methanol 85/15, Verdoppelung durch cis,trans-Isomerie) bestehende Endprodukt mit Schmp. 95-99 °C wird nach Umkristallisation aus Äthanol mit Hilfe von Säulenchromatographie (Kieselgel, Methylenchlorid/Methanol 95/5) in einzelne Fraktionen angereichert. Dabei konnten als polarer und weniger polarer Anteil die in Beispiel 11 und 8c beschriebenen cis,trans-2- bzw. 9-Chlor-11-cyanmethylen-6-(4-methyl-piperazin-1-yl)-morphantridin-Isomeren isoliert und charakterisiert werden.

Als weitere Fraktionen wurden die noch verbleibenden cis,trans-3- bzw. 8-Chlor-11-cyanmethylen-6-(4-methyl-piperazin-1-yl)-morphantridin-Isomeren angereichert. Gelbe Kristalle mit Schmp. 95-98 °C.

## Beispiel 3

cis,trans-4-Chlor-11-cyanmethylen-6-(4-methyl-piperazin-1-yl)-morphantridin · 1/2 $H_2O$

Herstellung analog Beispiel 1 : nach Säulenchromatographie (Kieselgel, Methylenchlorid/Methanol 95/5) gelbe Kristalle mit Schmp. 90-95 °C. Als Ausgangsmaterial diente das nach Lit. I (L. H. Werner et al., J. Med. Chem. *8*, 74 (1965)) hergestellte Ringerweiterungsprodukt aus 1-Chlor-anthrachinon, das durch Säulenchromatographie (Kieselgel, Methylenchlorid/Methanol 95/5) in die polare Komponente mit Schmp. 196-198 °C aufgetrennt war.

Die 4-Stellung des Chlors ist nicht gesichert. Die Wittig-Reaktion ergibt das 4-Chlor-11-cyanmethylen-5,6-dihydro-morphantridin-6-on mit Schmp. 231-232 °C.

## Beispiel 4

cis,trans-7-Chlor-11-cyanmethylen-6-(4-methyl-piperazin-1-yl)-morphantridin

Herstellung analog Beispiel 1 : gelbe Kristalle mit Schmp. 219-221 °C.

Als Ausgangsmaterial dient die unpolare Komponente des Monochlor-5,6-dihydro-morphantridin-6,11-dions (siehe Beispiel 9) mit schmp. 269-270 °C.

Die 7-Stellung des Chlors ist nicht gesichert. Die Carbonyl-Olefinierung ergibt das 7-Chlor-11-cyanmethylen-5,6-dihydro-morphantridin-6-on mit Schmp. 207-210 °C.

## Beispiel 5

cis,trans-2-Chlor-11-cyanmethylen-6-(4-methyl-piperazin-1-yl)-morphantridin · 1/2 $H_2O$

Herstellung analog Beispiel 1 : gelbe Kristalle mit Schmp. 157-162 °C.

Als Ausgangsmaterial dient 2-Chlor-5,6-dihydro-morphantridin-6,11-dion (E. Hardtmann u. H. Ott, J. Org. Chem. *34*, 2244-2248 (1969)).

Die Carbonyl-Olefinierung gemäß Beispiel 1b ergibt das 2-Chlor-11-cyanmethylen-5,6-dihydro-morphantridin-6-on mit Schmp. 270 °C.

## Beispiel 6

cis,trans-9-Fluor-11-cyanmethylen-6-(4-methyl-piperazin-1-yl)-morphantridin

a) 3,6 g (15,1 mM) 9-Amino-5,6-dihydro-morphantridin-6,11-dion (Beispiel 2a) wird in einer Mischung aus 100 ml Wasser und 100 ml conz. Salzsäure suspendiert. Nach Kühlen auf 0 bis 5 °C wird dann unter gutem Rühren eine Lösung von 1,06 g (15,1 mM) Natriumnitrit in 20 ml Wasser zugetropft. Die gelbe Reaktionsmischung wird anschließend 2 Stunden bei 0 bis 5 °C nachgerührt. Anschließend fügt man 100 ml 50 %ige Tetrafluorborsäure hinzu und läßt 1 Stunde bei 0 bis 5 °C nachrühren.

Der Niederschlag wird abgesaugt und mit reichlich Wasser ausgewaschen. Nach dem Trocknen an der Luft wird das Diazoniumtetrafluoroborat (4,8 g) in einem Zweihalskolben mit Rückflußkühler im leichten Stickstoffstrom erhitzt. Bei ca. 110 °C Badtemperatur setzt die Reaktion ein. Man erhöht nach Abklingen der Reaktion die Badtemperatur 15 Minuten lang noch auf 200 °C. Nach Abkühlen kocht man die Festkörper zur Reinigung dreimal in je 50 ml Methanol aus und saugt in der Hitze ab. Aus den Methanol-Mutterlaugen kristallisiert noch ein weiterer Teil des Produktes aus.

Man isoliert insgesamt 3,2 g cis,trans-5,6-Dihydro-9-fluor-morphantridin-6,11-dion vom Schmp. 250 bis 254 °C.

b) Weiterreaktion zum Endprodukt analog Beispiel 1 :

cis,trans-9-Fluor-11-cyanmethylen-5,6-dihydro-morphantridin-6-on nach Beispiel 1b : Schmp. 280-285 °C.

cis,trans-9-Fluor-11-cyanmethylen-6-(4-methyl-piperazin-1-yl)-morphantridin nach Beispiel 1a : Schmp. 120 bis 125 °C.

## Beispiel 7

a) cis,trans-3-Methyl-11-cyanmethylen-6-(4-methyl-piperazin-1-yl)-morphantridin

Herstellung analog Beispiel 1 : gelbe Kristalle mit Schmp. 192-200 °C. Die Position der Methyl-Gruppe in Stellung 3 ist durch Röntgenstrukturanalyse ermittelt.

Zur Trennung der cis,trans-Isomeren kristallisiert man das Isomerengemisch aus Methanol fraktioniert um : man isoliert als erste Fraktion stark angereichertes unpolares (Dünnschicht Kieselgel, Toluol/Methanol 85/15) Isomeres, das nochmals aus Methanol umkristallisiert wird. Dieses Isomere mit dem Schmp. 224 °C erweist sich nach Röntgenstrukturanalyse als cis-3-Methyl-11-cyanmethylen-6-(4-methyl-piperazin-1-yl)-morphantridin.

Die entsprechende polare trans-isomere Verbindung erhält man am besten durch fraktionierte Kristallisation des oben anfallenden Mutterlaugen-Rückstandes aus Cyclohexan ; das reine trans-3-Methyl-11-cyanmethylen-6-(4-methyl-piperazin-1-yl)-morphantridin schmilzt bei 193-195 °C.

Als Ausgangsmaterial dient das nacht Lit. I hergestellte Ringerweiterungsprodukt aus 2-Methylanthrachinon, das durch fraktionierte Kristallisation aus Toluol (schwerer lösliche Fraktion) und Umkristallisation aus Dimethylformamid nach Lit. I in 3-Methyl-5,6-dihydro-morphantridin-6,11-dion mit Schmp. 259 bis 263 °C separiert wird.

Die Carbonylolefinierung liefert cis,trans-3-Methyl-11-cyanmethylen-5,6-dihydro-morphantridin mit Schmp. 233-235 °C.

b) cis,trans-2-Methyl-11-cyanmethylen-6-(4-methyl-piperazin-1-yl)-morphantridin

Herstellung analog Beispiel 1 : gelbe Kristalle mit Schmp. 162-164 °C. Die Position der Methyl-Gruppe in Stellung 2 ist durch Röntgenstrukturanalyse ermittelt.

Zur Trennung der cis-trans-Isomeren kristallisiert man das Isomerengemisch aus Äthanol fraktioniert um : man isoliert als erste Fraktion stark angereichertes polares (Dünnschicht Kieselgel, Toluol/Methanol 85/15) Isomeres, das aus Äthanol umkristallisiert wird. Dieses Isomere mit dem Schmp. 183 °C erweist sich nach Röntgenstrukturanalyse als trans-2-Methyl-11-cyanmethylen-6-(4-methyl-piperazin-1-yl)-morphantridin · 1/2 $H_2O$.

Die entsprechende unpolare cis-isomere Verbindung erhält man am besten durch mehrmalige Säulenchromatographie (Kieselgel, Laufmittel Methylenchlorid/Methanol 98/2) des oben anfallenden Mutterlaugen-Rückstandes mit Schmp. 92-95 °C.

Als Ausgangsmaterial dient das in Toluol als leichter lösliche Fraktion (s. Beispiel 7a) erhaltene und durch Umkristallisation aus Dioxan/Ethanol 1 : 2 angereicherte 2-Methyl-5,6-dihydro-morphantridin-6,11-dion mit Schmp. 198-202 °C.

Durch weitere fraktionierte Kristallisation der Dioxan/Ethanol-Mutterlauge kann man auch eines der beiden restlichen noch vorliegenden 8- bzw. 9-Methyl-5,6-dihydro-morphantridin-6,11-dione anreichern.

Die Carbonyl-Olefinierung liefert das cis,trans-2-Methyl-11-cyanmethylen-5,6-dihydro-morphantridin-6-on mit Schmp. 228-230 °C.

## Beispiel 8 bis 10

Allgemeine Vorschrift zur Herstellung der Verbindungen 8 bis 10 zur Einführung der verschiedenen nukleophilen Alkylaminoreste in die 6-Stellung der 6-Chlormorphantridin-Derivate.

Das 6-Chlor-morphantridin wird mit 2 bis 5 Äquivalenten des Alkylamins AH versetzt und unter Stickstoff 3 bis 5 Stunden auf 110 °C erhitzt. Anschließend destilliert man bei Anwesenheit von leichter flüchtigen Aminen das überschüssige Nukleophil AH im Vakuum ab. In diesen Fällen nimmt man den Rückstand und ansonsten das Reaktionsgemisch in Eiswasser auf und extrahiert mehrmals mit Methylenchlorid. Die vereinigten Methylenchloridphasen werden nach Auswaschen mit Wasser getrocknet und eingeengt. Das verbleibende Rohprodukt wird entweder aus Ethanol unter Zusatz von Aktivkohle umkristallisiert oder (vor allem bei Anwesenheit der höhermolekularen Alkylamine) durch Säulenchromatographie (Kieselgel, Methylenchlorid/Methanol 95/5) gereinigt.

Folgende nach dieser Vorschrift hergestellten Verbindungen der Formel I seien hier aufgeführt :

8. cis,trans-11-Cyanmethylen-6-(4-ethyl-piperazin-1-yl)-morphantridin, Schmp. 86-90 °C :

Isomerentrennung von cis,trans-11-Cyanmethylen-6-(4-ethyl-piperazin-1-yl)-morphantridin :

Zur Trennung der cis,-trans-Isomeren kristallisiert man das Isomerengemisch fraktioniert aus Methanol um : als schwerer lösliche Fraktion kristallisiert zuerst das trans-Isomere (polare Komponente auf der Dünnschichtplatte : Kieselgel, Laufmittel Toluol/Methanol 85/15). Umkristallisieren aus Ethanol liefert das reine trans-Isomere mit Schmp. 181 bis 183 °C.

Säulenchromatographie (Kieselgel, Methylenchlorid/Methanol 95/5) des Mutterlaugenrückstandes reinigt auch das unpolarere cis-Isomere mit Schmp. 138 bis 140 °C.

9. cis,trans-11-Cyanmethylen-6-homopiperazin-1-yl-morphantridin · HCl · $H_2O$, Schmp. 175-178 °C.

10. cis,trans-11-Cyanmethylen-6-(2-piperidin-1-yl-ethylamino)-morphantridin · 1/2 $H_2O$, Schmp. 83-85 °C :

Isomerentrennung von cis,trans-11-Cyanmethylen-6-(2-piperidin-1-yl-ethylamino)-morphantridin :

Die Trennung der cis,trans-Isomeren kann mit Hilfe von Säulenchromatographie vorgenommen werden (Kieselgel, Methylenchlorid/Methanol 95/5) : das cis-Isomere (unpolare Komponente auf der Dünnschichtplatte : Kieselgel, Laufmittel Toluol/Methanol 85/15) fällt in Form von gelblichen Kristallen mit Schmp. 76-78 °C, das polarere trans-Isomere schmilzt bei 103-106 °C.

Beispiel 11

cis-trans-11-Cyanmethylen-6-(4-methyl-4-oxy-piperazin-1-yl)-morphantridin · 2 $H_2O$

3,0 g (9,1 mM) cis,trans-11-Cyanmethylen-6-(4-methyl-piperazin-1-yl)-morphantridin wird in 100 ml heißem Ethanol gelöst und mit 1,5 ml 30 %igem wasserstoffperoxid versetzt. Nach 5-stündigem Rückflußkochen zerstört man das überschüssige Wasserstoffperoxid mit Hilfe eines kleinen Platinblechs, das in das Reaktionsgemisch geworfen wird, durch 2-stündiges Rückflußkochen. nach Filtrieren engt man das Reaktionsgemisch ein und reinigt das erhaltene N-Oxid durch Säulenchromatographie (Kieselgel, Laufmittel Methylenchlorid/Methanol 95/5). Man isoliert 2,5 g (80 %) gelbe Kristalle mit Schmp. 141-148 °C.

Zur Trennung der cis-trans-Isomeren kristallisiert man das Isomerengemisch aus wenig Methylenchlorid fraktioniert um : man isoliert als erste Fraktion stark angereichertes unpolares (Dünnschicht, Kieselgel, Toluol/Methanol 85/15) Isomeres, das aus wenig Ethanol umkristallisiert wird. Aus Analogiegründen zu den vorher beschriebenen cis,trans-Isomeren-Analysen ordnet man diese Isomere mit dem Schmp. 241 °C der cis-Reihe zu.

Die entsprechende polare trans-isomere Verbindung mit Schmp. 169 °C erhält man durch Säulenchromatographie (Kieselgel, Laufmittel Methylenchlorid/Methanol 95/5) des oben anfallenden Mutterlaugen-Rückstandes.

Vorteilhaft stellt man beide Isomeren direkt durch Oxidation der beiden nach Beispiel 1 getrennten cis- und trans-Isomeren 11-Cyanmethylen-6-(4-methyl-piperazin-1-yl)-morphantridine (Beispiel 1) nach der obigen Vorschrift dar : während der Oxidation findet keine cis,trans-Isomerisierung statt.

Beispiel 12

Nach der obigen allgemeinen Vorschrift (Beispiel 8-10) wird weiterhin erhalten :

12. cis,trans-11-Cyanmethylen-6-(N'-methyl-homopiperazin-1-yl)- morphantridin · 0,75 $H_2O$, Schmp. 73-80 °C.

Formulierungsbeispiele, die in üblicher Weise hergestellt werden :

1. Tabletten

| | |
|---|---|
| a) Ein Wirkstoff der Formel I | 5 mg |
| Lactose | 200 mg |

| | |
|---|---|
| Methylcellulose | 15 mg |
| Maisstärke | 50 mg |
| Talkum | 11 mg |
| Magnesiumstearat | 4 mg |
| | 285 mg |

| | |
|---|---|
| b) Ein Wirkstoff der Formel I | 20 mg |
| Lactose | 178 mg |
| Avicel | 80 mg |
| Polywachs 6 000 | 20 mg |
| Magnesiumstearat | 2 mg |
| | 300 mg |

| | |
|---|---|
| c) Verbindung der Formel I | 50 mg |
| Polyvinylpyrrolidon (mittel. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50 °C getrocknet. Dieses Granulat wird mit Polyäthylenglykol (mittl. M.G. 4 000), Hydroxypropylmethylcellulose, Talkum und Magnesiumstearat vermischt und zu Tabletten à 280 mg verpreßt.

2. Beispiel für Dragees

| | |
|---|---|
| Verbindung der Formel I | 3 mg |
| Lactose | 90 mg |
| Maisstärke | 60 mg |
| Polyvinylpyrrolidon | 6 mg |
| Magnesiumstearat | 1 mg |
| | 160 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50 °C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Kapselformulierung

| | |
|---|---|
| Verbindung der Formel I | 5,0 mg |
| Magnesiumstearat | 2,0 mg |
| Milchzucker | 19,3 mg |

4. Injektionslösung

| | |
|---|---|
| Verbindung der Formel I | 10 mg |
| Natriumchlorid destilliertes Wasser, q.s. auf 1,0 ml | 9 mg |

**Ansprüche**

1. 6-substituierte 11-Alkylen-morphantridine der allgemeinen Formel I

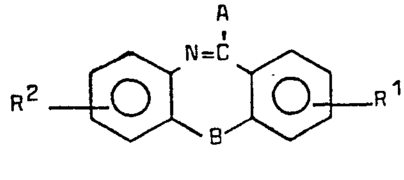

(I)

in der $R^1$ und $R^2$ Wasserstoff, Fluor, Chlor oder Methyl bedeuten, A für 4-Methyl-piperazin-1-yl, 4-Ethyl-piperazin-1-yl, 4-Methyl-4-oxy-piperazin-1-yl, N'-Methyl-homopiperazin-1-yl oder 2-Piperidin-1-yl-ethylamino steht und B einen Alkylenrest der Formel

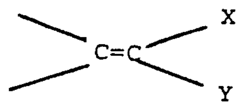

in der X ein Wasserstoffatom und Y eine Cyanogruppe bedeuten, ihre reinen cis- und trans-Isomeren und ihre physiologisch verträglichen Säureadditionssalze.

2. cis,trans-Gemisch von 11-Cyanmethylen-6-(4-methyl-piperazin-1-yl)-morphantridin nach Anspruch 1 mit einem Schmelzpunktsbereich von 148-150 °C.

3. cis-11-Cyanmethylen-6-(4-methyl-piperazin-1-yl)-morphantridin nach Anspruch 1.

4. trans-11-Cyanmethylen-6-(4-methyl-piperazin-1-yl)-morphantridin nach Anspruch 1.

5. cis, trans-Gemisch von 11-Cyanmethylen-6-(4-methyl-4-oxy-piperazin-1-yl)-morphantridin nach Anspruch 1 mit einem Schmelzpunktsbereich von 141-148 °C.

6. cis-11-Cyanmethylen-6-(4-methyl-4-oxy-piperazin-1-yl)-morphantridin nach Anspruch 1.

7. trans-11-Cyanmethylen-6-(4-methyl-4-oxy-piperazin-1-yl)-morphantridin nach Anspruch 1.

8. cis,trans-Gemisch von 11-Cyanmethylen-2-methyl-6-(4-methyl-piperazin-1-yl)-morphantridin nach Anspruch 1 mit einem Schmelzpunktsbereich von 162-164 °C.

9. cis-trans-Gemisch von 11-Cyanmethylen-3-methyl-6-(4-methyl-piperazin-1-yl)-morphantridin nach Anspruch 1 mit einem Schmelzpunktsbereich von 192-200 °C.

10. cis-11-Cyanmethylen-3-methyl-6-(4-methyl-piperazin-1-yl)-morphantridin nach Anspruch 1.

11. trans-11-Cyanmethylen-3-methyl-6-(4-methyl-piperazin-1-yl)-morphantridin nach Anspruch 1.

12. cis,trans-Gemisch von 11-Cyanmethylen-6-(4-ethyl-piperazin-1-yl)-morphantridin nach Anspruch 1 mit einem Schmelzpunktsbereich von 86-90 °C.

13. trans-11-Cyanmethylen-6-(4-ethyl-piperazin-1-yl)-morphantridin nach Anspruch 1.

14. cis,trans-Gemisch von 11-Cyanmethylen-6-(N'-methyl-homopiperazin-1-yl)-morphantridin nach Anspruch 1 mit einem Schmelzpunktsbereich von 73-80 °C.

15. cis,trans-Gemisch von 11-Cyanmethylen-6-(2-piperidin-1-yl-ethylamino)-morphantridin nach Anspruch 1 mit einem Schmelzpunktsbereich von 83-85 °C.

16. Verfahren zur Herstellung von Verbindung der Formel I

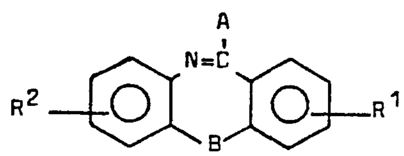

(I)

in der $R^1$ und $R^2$ Wasserstoff, Fluor, Chlor oder Methyl bedeuten, A für 4-Methyl-piperazin-1-yl, 4-Ethyl-piperazin-1-yl, 4-Methyl-4-oxy-piperazin-1-yl, N'-Methyl-homopiperazin-1-yl oder 2-Piperidin-1-yl-ethylamino steht und B einen Alkylenrest der Formel

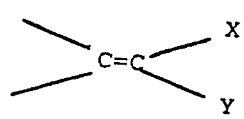

in der X ein Wasserstoffatom und Y eine Cyanogruppe bedeuten, ihre reinen cis- und trans-Isomeren und ihre physiologisch verträglichen Säureadditionssalze, dadurch gekennzeichnet, daß man eine Verbindung der Formel VII

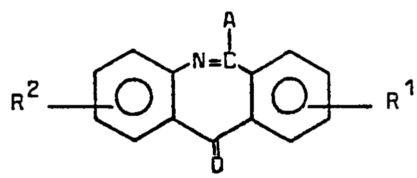

(VII)

in der $R^1$, $R^2$ und A die für Formel I angegebenen Bedeutungen haben, mit einer Verbindung der Formel V

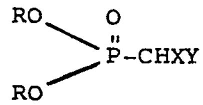

(V)

in der X, Y die im Rest B der Formel I für X und Y angegebenen Bedeutungen haben und R Alkylreste mit 1 bis 3 C-Atomen bedeuten, unter den Bedingungen der Wittig-Horner-Reaktion umsetzt, gegebenenfalls in das reine cis- und trans-Isomere trennt und gegebenenfalls im Falle eines 4-Methyl-piperazin-1-yl-restes die erhaltene Verbindung in das N-Oxid und/oder Säureadditionssalz einer physiologisch verträglichen Säure überführt oder daß man eine Verbindung der Formel II

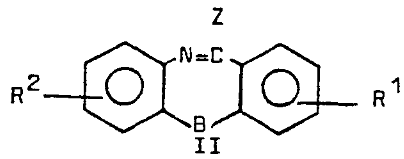

II

in der $R^1$, $R^2$ und B die für Formel I angegebenen Bedeutungen haben und Z eine nukleofuge Abgangsgruppe darstellt, mit einem Nukleophil AH, in dem A die für Formel I angegebenen Bedeutungen hat, umsetzt, gegebenenfalls in das reine cis- und trans-Isomere trennt und gegebenenfalls im Falle eines 4-Methylpiperazin-1-yl-restes die erhaltene Verbindung in das N-Oxid und/oder in das Säureadditionssalz einer physiologisch verträglichen Säure überführt.

17. Therapeutisches Mittel, gekennzeichnet durch einen Gehalt an einer Verbindung der Formel I, deren reines cis- oder trans-Isomeres oder deren pharmakologisch verträgliches Säureadditionssalz als Wirkstoff neben üblichen Träger- und Verdünnungsmitteln.

## Claims

1. A 6-substituted 11-alkylene-morphantridine of the general formula I

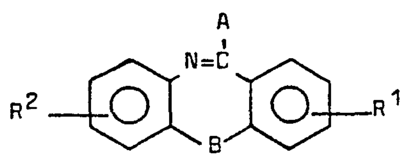

(I)

where $R^1$ and $R^2$ are hydrogen, fluorine, chlorine or methyl, A is 4-methyl-piperazin-1-yl, 4-ethyl-piperazin-1-yl, 4-methyl-4-oxy-piperazin-1-yl, N'-methyl-homopiperazin-1-yl or 2-piperidin-1-yl-ethylamino, and B is alkylene of the formula

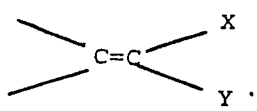

where X is hydrogen, and Y is cyano, its pure cis- and trans-isomers and its physiologically tolerated acid addition salts.

2. A cis,trans-isomer mixture of 11-cyanomethylene-6-(4-methyl-piperazin-1-yl)-morphanthridine, as claimed in claim 1, having a melting point of from 148 to 150 °C.

3. cis-11-Cyanomethylene-6-(4-methyl-piperazin-1-yl)-morphanthridine as claimed in claim 1.

4. trans-11-Cyanomethylene-6-(4-methyl-piperazin-1-yl)-morphanthridine as claimed in claim 1.

5. A cis,trans-isomer mixture of 11-cyanomethylene-6-(4-methyl-4-oxy-piperazin-1-yl)-morphanthridine, as claimed in claim 1, having a melting point of from 141 to 148 °C.

6. cis-11-Cyanomethylene-6-(4-methyl-4-oxy-piperazin-1-yl)-morphanthridine as claimed in claim 1.

7. trans-11-Cyanomethylene-6-(4-methyl-oxy-piperazin-1-yl)-morphanthridine as claimed in claim 1.

8. A cis,trans-isomer mixture of 11-cyanomethylene-2-methyl-6-(4-methyl-piperazin-1-yl)-morphanthridine, as claimed in claim 1, having a melting point of 162 to 164 °C.

9. A cis,trans-isomer mixture of 11-cyanomethylene-3-methyl-6-(4-methyl-piperazin-1-yl)-morphanthridine, as claimed in claim 1, having a melting point of from 192 to 200 °C.

10. cis-11-Cyanomethylene-3-methyl-6-(4-methyl-piperazin-1-yl)-morphanthridine as claimed in claim 1.

11. trans-11-Cyanomethylene-3-methyl-6-(4-methyl-piperazin-1-yl)-morphanthridine as claimed in claim 1.

12. A cis,trans-isomer mixture of 11-cyanomethylene-6-(4-ethyl-piperazin-1-yl)-morphanthridine, as claimed in claim 1, having a melting point of from 86 to 90 °C.

13. trans-11-Cyanomethylene-6-(4-ethyl-piperazin-1-yl)-morphanthridine as claimed in claim 1.

14. A cis,trans-isomer mixture of 11-cyanomethylene-6-(N'-methyl-homopiperazin-1-yl)-morphanthridine, as claimed in claim 1, having a melting point of from 73 to 80 °C.

15. A cis,trans-isomer mixture of 11-cyanomethylene-6-(2-piperidin-1-yl-ethyl-amino)-morphanthridine, as claimed in claim 1, having a melting point of from 83 to 85 °C.

16. A process for the preparation of a compound of the formula I

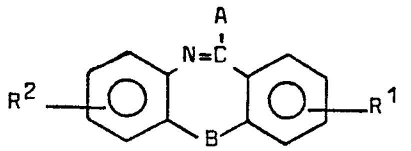

(I)

where $R^1$ and $R^2$ are hydrogen, fluorine, chlorine or methyl, A is 4-methyl-piperazin-1-yl, 4-ethyl-piperazin-1-yl, 4-methyl-4-oxy-piperazin-1-yl, N'-methyl-homopiperazin-1-yl or 2-piperidin-1-yl-ethylamino, and B is alkylene of the formula

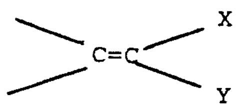

where X is hydrogen, and Y is cyano, its pure cis- and trans-isomers and its physiologically tolerated acid addition salts, wherein a compound of the formula VII

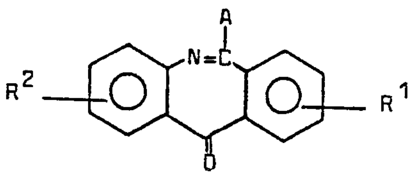

(VII)

where $R^1$, $R^2$ and A have the meanings given for formula I, is reacted with a compound of the formula V

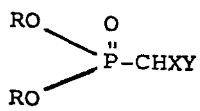

(V)

where X and Y have the meanings given for X and Y in the radical B of formula I and the R's are alkyls of 1 to 3 carbon atoms, under the conditions of a Wittig-Horner reaction, and, if desired, the product is separated into the pure cis- and trans-isomers and, if desired, in the case of a 4-methyl-piperazin-1-yl radical the resulting compound is converted into the N-oxide and/or an addition salt with a physiologically tolerated acid, or a compound of the formula II

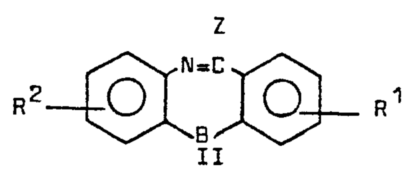

II

where $R^1$, $R^2$ and B have the meanings given for formula I, and Z is a nucleofugic leaving group, is reacted with a nucleophilic agent AH, where A has the meanings given for formula I, and, if desired, the product is separated into the pure cis- and trans-isomers and, if desired, in the case of a 4-methyl-piperazin-1-yl radical the resulting compound is converted into the N-oxide and/or an addition salt with a physiologically tolerated acid.

17. A therapeutic agent which contains a compound of the formula I, or its pure cis- or trans-isomer, or a pharmacologically tolerated acid addition salt of any of these, as the active compound, together with conventional carriers and diluents.

**Revendications**

1. 11-alkylène-morphantridine substituée en 6 de formule générale

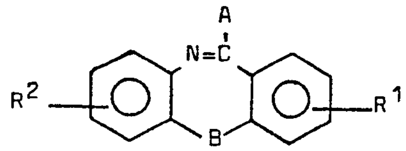

(I)

dans laquelle $R^1$ et $R^2$ représentent hydrogène, fluor, chlore ou méthyle, A représente 4-méthyl-pipérazin-1-yle, 4-éthyl-pipérazin-1-yle, 4-méthyl-4-oxy-pipérazin-1-yle, N'-méthyl-homopipérazin-1-yle, ou 2-pipéridin-1-yl-éthylamino, et B un reste alkylène de formule

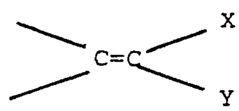

dans laquelle X représente un atome d'hydrogène et Y un groupe cyano, leurs cis- et trans-isomères purs et leurs sels d'addition d'acide compatibles physiologiquement.

2. Mélange cis,trans de 11-cyanométhylèn-6-(4-méthyl-pipérazin-1-yl)-morphantridine selon la revendication 1, ayant un point de fusion situé dans la zone de 148 à 150 °C.

3. Cis-11-cyanométhylèn-6-(4-méthyl-pipérazin-1-yl)-morphantridine selon la revendication 1.

4. Trans-11-cyanométhylèn-6-(4-méthyl-pipérazin-1-yl)-morphantridine selon la revendication 1.

5. Mélange cis, trans de 11-cyanométhylèn-6-(4-méthyl-4-oxy-pipérazin-1-yl)-morphantridine selon la revendication 1, d'un point de fusion situé dans la zone de 141-148 °C.

6. Cis-11-cyanométhylèn-6-(4-méthyl-4-oxy-pipérazin-1-yl)-morphantridine selon la revendication 1.

7. Trans-11-cyanométhylèn-6-(4-méthyl-4-oxy-pipérazin-1-yl)-morphantridine selon la revendication 1.

8. Mélange cis, trans de 11-cyanométhylèn-2-méthyl-6-(4-méthyl-pipérazin-1-yl)-morphantridine selon la revendication 1 d'un point de fusion situé dans la zone de 162-164 °C.

9. Mélange cis, trans de 11-cyanométhylèn-3-méthyl-6-(4-méthyl-pipérazin-1-yl)-morphantridine selon la revendication 1, d'un point de fusion situé dans la zone de 192-200 °C.

10. Cis-11-cyanométhylèn-3-méthyl-6-(4-méthyl-pipérazin-1-yl)-morphantridine selon la revendication 1.

11. Trans-11-cyanométhylèn-3-méthyl-6-(4-méthyl-pipérazin-1-yl)-morphantridine selon la revendication 1.

12. Mélange cis, trans de 11-cyanométhylène-6-(4-éthyl-pipérazin-1-yl)-morphantridine selon la revendication 1, d'un point de fusion compris dans la zone de 86-90 °C.

13. Trans-11-cyanométhylèn-6-(4-éthyl-pipérazin-1-yl)-morphantridine selon la revendication 1.

14. Mélange cis, trans de 11-cyanométhylèn-6-(N'-méthyl-homopipérazin-1-yl)-morphantridine selon la revendication 1, d'un point de fusion compris dans la zone de 73-80 °C.

15. Mélange cis, trans de 11-cyanométhylèn-6-(2-pipéridin-1-yl-éthylamino-morphantridine selon la revendication 1, d'un point de fusion compris dans la zone de 83-85 °C.

16. Procédé de préparation de composés de formule I

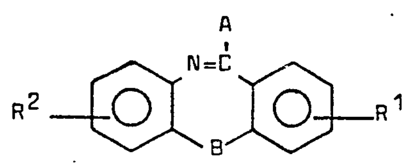

(I)

dans laquelle $R^1$ et $R^2$ représentent hydrogène, fluor, chlore ou méthyle, A représente 4-méthyl-pipérazin-1-yle, 4-éthyl-pipérazin-1-yle, 4-méthyl-4-oxy-pipérazin-1-yle, N'-méthyl-homopipérazin-1-yle ou 2-pipéridin-1-yl-éthylamino, et B un reste alkylène de formule

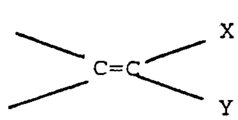

dans laquelle X représente un atome d'hydrogène et Y un groupe cyano, leurs cis et trans-isomères purs et leurs sels d'addition d'acide compatibles physiologiquement, caractérisé par le fait qu'on fait réagir un composé de formule VII

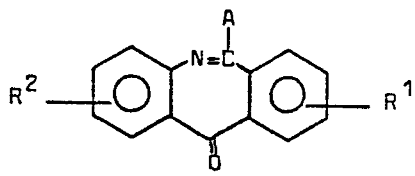

(VII)

dans laquelle $R^1$, $R^2$ et A ont les significations données pour la formule I, avec un composé de formule V

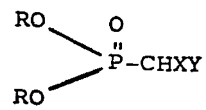

(V)

dans laquelle X, Y ont les significations données pour X, Y dans le reste B de la formule I, et R représente un reste alkyle ayant 1 à 3 atomes C, dans les conditions de la réaction de Wittig-Horner, on sépare éventuellement en les isomères cis- et trans-, et on transforme éventuellement, dans le cas d'un reste 4-méthyl-pipérazin-1-yle, le composé obtenu en le N-oxyde et/ou le sel d'addition d'acide d'un acide compatible physiologiquement, ou on fait réagir un composé de formule II

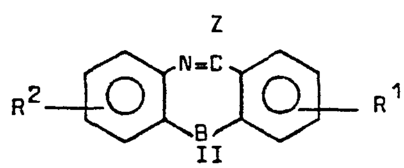

II

dans laquelle $R^1$, $R^2$ et B ont les significations données pour la formule I et Z représente un groupe de départ nucléofuge, avec un nucléophile AH, où A a la signification donnée pour la formule I, on sépare éventuellement en les isomères cis et trans, et on transforme éventuellement, dans le cas d'un reste 4-méthyl-pipérazin-1-yle, le composé obtenu en le N-oxyde et/ou le sel d'addition d'acide d'un acide compatible physiologiquement.

17. Agent thérapeutique, caractérisé par le fait qu'il contient un composé de formule I, son isomère cis- ou trans-, pur ou son sel d'addition d'acide, compatible physiologiquement, comme principe actif, à côté des véhicules et diluants usuels.